## Europäisches Patentamt
## European Patent Office
## Office européen des brevets

(19)

(11) Publication number: **0 032 293**
**A2**

(12) # EUROPEAN PATENT APPLICATION

(21) Application number: **80304378.5**

(22) Date of filing: **04.12.80**

(51) Int. Cl.³: **C 12 P 19/06**
**C 08 B 37/00, C 09 D 7/00**
**D 06 P 1/48, C 11 D 3/22**

(30) Priority: **20.12.79 GB 7943871**

(43) Date of publication of application:
**22.07.81 Bulletin 81/29**

(84) Designated Contracting States:
**BE DE FR IT LU NL**

(71) Applicant: **TATE & LYLE PATENT HOLDINGS LIMITED**
**5th Floor, Triningham Building Front Street**
**Hamilton 5(BM)**

(84) Designated Contracting States:
**DE FR IT LU NL**

(71) Applicant: **TALRES DEVELOPMENT (N.A.) N.V.**
**Breedestraat 39C**
**Curaçao, Netherlands Antilles(NL)**

(84) Designated Contracting States:
**BE**

(72) Inventor: **Pace, Gary William c/o Dr. D.W.Fewkes**
**Tate & Lyle Ltd 24/26 Queens Road**
**Reading RG1 4AU Berkshire(GB)**

(72) Inventor: **Coote, Stephen Donald John**
**16, Rawlinson Road Hesketh Park**
**South Port Merseyside(GB)**

(74) Representative: **Ablewhite, Alan James et al,**
**MARKS & CLERK 57 60 Lincoln's Inn Fields**
**London WC2A 3LS(GB)**

(54) Xanthan gum, and method of producing it by fermentation.

(57) Novel xanthan gums with an intrinsic viscosity $[\eta]$ of less than 14 dl/g can be produced by effecting a *xanthomonas* fermentation at a broth temperature of at least 31°C.

EP 0 032 293 A2

- 1 -

"XANTHAN GUM".

TITLE MODIFIED
see front page

The present invention relates to xanthan gum.

Xanthan gum is an exocellular polysaccharide produced on an industrial scale by fermentation of micro-organisms of the genus Xanthomonas. The polysaccharide is generally considered to comprise a $\beta$-(1→4)-linked D-glucosyl backbone with side-chains of D-mannose and D-glucuronic acid. The D-glucose: D-mannose : D-glucuronic acid molar ratio is typically around 2:2:1, but this ratio is subject to some variation from product to product.

Acetyl and pyruvate units are also usually attached to the chain, with the D-glucose : D-mannose : D-glucuronic acid : acetyl : pyruvate ratio normally being about 2:2:1:1:0.5. However, other xanthan gums are known which contain higher or lower proportions of acetyl and/or pyruvate. For an example of such other gums, see UK Patent Specification No. 2008600 where it is said that a deacetylated pyruvate-free xanthan has been made.

Xanthan gum, as a material, is characterized by its excellent stability in the presence of acids, bases and salts, It is also stable in the presence of cellulase enzymes. The stability of xanthan is an important feature which has contributed to its adoption in a wide variety of products and uses, notably in foods and for oil recovery.

Xanthan gum available to-day is further characterized by its particular rheological properties. While there are some differences depending on the source, the xanthan gums commercially available at the present time all have essentially the same high thickening power. In other words, the gum concentration required to give a particular apparent viscosity under standardized conditions is substantially the same for all the gums. Moreover, the xanthan gums on the market to-day all give extremely pseudoplastic solutions : under conditions of low shear the apparent viscosity is very high, while under high shear the apparent viscosity is very low.

For some end-uses eg paints or printing pastes, a thickening agent having less pseudoplasticity is often required. Depending on the flow properties which may be desired, it is advantageous to be able to choose between a relatively low concentration of a grade of thickening agent with high viscosity, and a high concentration of a grade of thickening agent with low viscosity.

This choice is not currently possible with the commercially available xanthan gums, since they have essentially the same rheological properties. To some extent, the lack of choice of viscosity grades explains why xanthan gum has not been wholly adopted as a suitable alternative to hydroxyethylcellulose and the other water-soluble polymers which are available in different viscosity grades for use as thickening agents.

The water-soluble polymers used as thickening agents to provide different grades of viscosity suffer from poor stability and often give products with a shorter-than-desired shelf life.

There is thus a need for a thickening agent having the stability inherent in xanthan gums, but with a lower pseudoplasticity than the commercially available xanthan.

We have now unexpectedly found that fermentations with Xanthomonas spp can be performed in such a manner as to give a xanthan gum with less pseudoplasticity. Our novel xanthan gum thus represents a low viscosity grade of xanthan.

In accordance with the present invention there is provided a xanthan gum with an intrinsic viscosity $[\eta]$ of less than 14 dl/g.

- 4 -

Whereas previous xanthan gums have an intrinsic viscosity of above 15 dl/g, often 20 to 25 dl/g, the present gums have an intrinsic viscosity of less than 14 dl/g, preferably less than 12.5 dl/g.

Intrinsic viscosity $[\eta]$ can be measured by the following procedure.

1)      A series of solutions of the gum are prepared in aqueous 0.1 $\underline{M}$ KCl solution as solvent over a range of concentrations not exceeding 0.02% w/v.

2)      The flow time $\underline{t}$ of each solution and of pure solvent is measured in a capillary viscometer at $25.00 \pm 0.05^{\circ}C$. The liquids are filtered through glass sinter or millipore filter ( 5 µm pore size) before the viscometry.

3)      The relative viscosity for each solution is determined in accordance with the relationship:

$$\eta_r = \frac{\eta_{\text{solution}}}{\eta_{\text{solvent}}} = \frac{t_{\text{solution}}}{t_{\text{solvent}}} \times \frac{\rho_{\text{solvent}}}{\rho_{\text{solution}}}$$

where the ratio of densities $\rho$ solvent/ $\rho$ solution is usually taken to be unity at low solute concentrations.

4).     The reduced viscosity $\eta_{sp}$ for each solution is determined in accordance with the relationship:

$$\frac{\eta_{sp}}{C} = \frac{\eta_r - 1}{C}$$

5).     Reduced viscosity is then plotted against concentration. The plot should be linear; intrinsic viscosity $[\eta]$ is then given by the intercept on the ordinate at $C = 0$.

An alternative plot of $(1/C) \ln \eta_r$ versus C is also linear with the same intercept $[\eta]$ but has a smaller slope. Thus $(1/C) \ln \eta_r$ is much less concentration - dependent than the reduced viscosity.

Evaluation of $(1/C) \ln \eta_r$ at a single low concentration is close to the actual intrinsic viscosity value and can be determined more quickly. When $(1/C) \ln \eta_r$ is determined at $C = 0.01\%$ (usually using 1% NaCl as solvent but this difference in solvents is regarded as insignificant), it is called the inherent viscosity $\eta_{\text{inh.}}$

The gums of the present invention can be prepared by a modification of the conventional procedures involving fermentation with a micro-organism of the genus Xanthomonas. As described in the patent literature (eg US Patents 3000790, 3271267, 3328262, 3391060 and 3433708) and in other technical literature (eg Biotechnology and Bioengineering, vol VIII (1966) 511; ibid, vol XIV (1972) 23; ibid vol XV (1973) 225 and ibid, vol XX (1978) 1003) xanthan gum can be produced by fermentation of a carbohydrate-containing broth with X. campestris or other micro-organism of the same genus. In practice the broth is held at around $28^{\circ}C$ so as to maximise yields.

Unexpectedly we have now found that the novel gums of the present invention are produced in a modified fermentation method which also forms part of this invention.

In accordance with the present invention, there is provided a method for producing a xanthan gum by fermentation of a carbohydrate-containing broth using a micro-organism of the genus Xanthomonas characterized in that the fermentation is effected at a broth temperature of at least $31^{\circ}C$ and a xanthan gum with an intrinsic viscosity of less than 14 dl/g is produced.

The preferred temperature for the present process is around 35$^\circ$C, with 32 - 38$^\circ$C representing a preferred range.

Apart from the difference in broth temperature, and the unexpected concomitant production of the gum with reduced viscosity, the present process can be performed in a similar manner to the known fermentations using <u>Xanthomonas</u> <u>spp.</u> Further details are to be found in the extensive patent and other literature, and it is unnecessary to mention all the possibilities in this specification.

Suitably, the present process is carried out as a batch or continuous fermentation of broth containing a carbohydrate, such as glucose, an inorganic and/or organic source of assimalable nitrogen, other nutrients including trace elements, and any necessary essential growth factors. The micro-organism will usually be <u>X.</u> <u>campestris</u>, though others can be employed to advantage. After the fermentation the gum can be isolated by a known procedure such as precipitation and filtration. Isolation of the gum is however not essential.

The xanthan gums of the present invention can be used in water-based paints, textile printing pastes, cleaning compositions and in many other products. The present gums have the advantage that they are less pseudoplastic than conventional xanthan gums, and can be used at a higher concentration to provide the same viscosity.

The present invention is illustrated by the following non-limiting examples. A comparative example is given first:

Comparative Example 1 : Conventional xanthan gum

A laboratory stock of Xanthomonas campestris derived from the publically available strain X. campestris ATCC 13951 was grown batchwise in a 10 litre capacity continuously stirred tank fermenter. 8 l of sterilized fermentation medium having the composition shown in Table 1 was used.

TABLE 1

| Component | Amount g/l |
|---|---|
| Glucose(anhydrous) | 40.0 |
| $(NH_4)_2SO_4$ | 2.0 |
| $KH_2PO_4$ | 1.0 |
| $MgSO_4 7H_2O$ | 0.2 |
| Yeast Extract | 4.0 |

Two curved bladed impellers of 9.7 cm diameter were set at 6 cm and 15 cm from the base of the fermenter, and four baffles were installed around the inside of the tank wall to aid mixing. Air was introduced below the lower impeller. The fermenter was fitted with automatic pH control for metering in 2M NaOH to maintain the pH at the desired value.

The medium in the fermenter was inoculated with 400 ml of a culture grown in shake flasks in " MYGP" liquid medium (g/l Malt extract 3.0; yeast extract 3.0; glucose 10.0; peptone 5.0).

-10-

The fermentation parameters were set to be as shown in Table 2:

TABLE 2

| pH | 7.0 |
|----|-----|
| Temperature | 30°C |
| Impeller speed | 775 r.p.m. |
| Air flow | 4 litres. m⁻¹ |

Foaming was controlled, as necessary, by addition of polypropylene glycol 2025 (BDH Chemical Co. Ltd., Poole, Dorset).

The culture in the fermenter was monitored by measurement of:-

(i) pH

(ii) Total precipitable matter (TPM-the precipitate from addition of 1.5 volumes of isopropanol to 1 part by weight of fermenting medium was collected by filtration and dried under an infra-red lamp for 45 minutes.

(iii) Cell density, by optical density at 540 nm ("OD 540") (iv) Residual glucose, by a glucose oxidase method.

- 11 -

(v) Viscosity of culture broth, using a Wells Brookfield cone and plate microviscometer at $25^\circ$C.

The data for the culture was as given in Table 3. Percentages in this and the following Tables are by weight.

TABLE 3

| | |
|---|---|
| Initial glucose | 41.6 g/l |
| Final glucose | 0.1 g/l |
| OD 540 (1/10 dilution by wt) | 0.36 |
| TPM | 23.9 g/l |
| Yield $(\frac{TPM}{glucose\ used} \times 100)$ | 58% |
| Consistency index of final broth, K | 31,000 m Pa.s$^n$. |

At the completion of the fermentation the xanthan gum was precipitated from the culture broth by mixing with 2 volumes of isopropanol. The precipitate and bacterial cells were recovered by filtration, squeezed to remove excess liquid and freeze-dried.

The dried xanthan gum was analysed for chemical composition and the viscosities of 1% solutions of the gum in two solvents (distilled water and 1% NaCl solution) were measured. The results were as given in Table 4.

TABLE 4

| Moisture | 2.6% |
|---|---|
| Ash(sulphated) | 12.8% |
| Nitrogen | 2.5% |
| Pyruvate | 5.1% |
| Consistency index in dist $H_2O$ K | $5,000 \times mPas^n$ |
| n | 0.22 |
| Consistency index in NaCl solution K | $5,500 \times mPa.s^n$ |
| n | 0.24 |

Example 1: Low viscosity xanthan gum

The fermentation was repeated except that the temperature of the medium was maintained at $35^{\circ}C$.

The data analogous to those of Table 3 were as shown in Table 5.

TABLE 5

| Initial glucose | 41.5 g/l |
|---|---|
| Final glucose | 0.4 g/l |
| TPM | 21.1 g/l |
| Final OD 540 (1/10 dilution) | 0.44 |
| Yield | 52% |

The xanthan gum was recovered as in the Comparative Example and analysed extensively. The results of the tests are given in Table 6.

TABLE 6

| Moisture | 9.4% |
|---|---|
| Ash | 11.7% |
| Nitrogen | 2.3% |
| Pyruvate | 3.8% |
| Acetate | 5.4% |
| $Na^+$ | 3.37% |
| $K^+$ | 0.73% |
| Intrinsic viscosity | 11.5dl/g |
| Molecular weight (from intrinsic viscosity) | $9.8 \times 10^5 - 2.5 \times 10^6$ |
| Equivalent weight | 697 |
| Uronic acid | 19.4% |
| Hexoses- glucose: mannose | 1.05:1 |
| Infra-red spectrum | superimposable on that of commercial xanthan gum |
| Consistency index in dist. $H_2O$     K | 540 $mPa.s^n$ |
| n | 0.48 |

cont/.....

- 14 -

| Consistency index in 1% NaCl | $1380 \ mPa.s^n$ |
| K | |
| n | 0.37 |

The data shows that the material is a xanthan gum with an intrinsic viscosity lower than that for conventional material.

Values of $[\eta]$ or $\eta$inh for seven commercial samples of xanthan gum were determined for comparison with those of the present novel gums. The values in dl/g are summarized in Table 7:

Table 7 : Viscosities of Commercial Samples.

| | $[\eta]$ | $\eta$ inh |
|---|---|---|
| Mean | 25.0 | 23.9 |
| Lowest | 20.7 | 19.9 |
| Highest | 28.3 | 25.5 |

The intrinsic viscosity for the product of the Example is 11.5 dl/g and the inherent viscosity is 11.4 dl/g.

The inherent and intrinsic viscosities for the product of the Example are markedly different to those for the known products.

There is also a marked difference in the pseudoplasticity of the present product when compared with the conventional gums.

In order to demonstrate this effect, the variation of viscosity with shear ४ was determined for a 1% solution of a product in accordance with the present invention and for a 0.3% solution of a conventional product. The difference in concentrations was selected so as to give solutions with comparable viscosities at no shear.

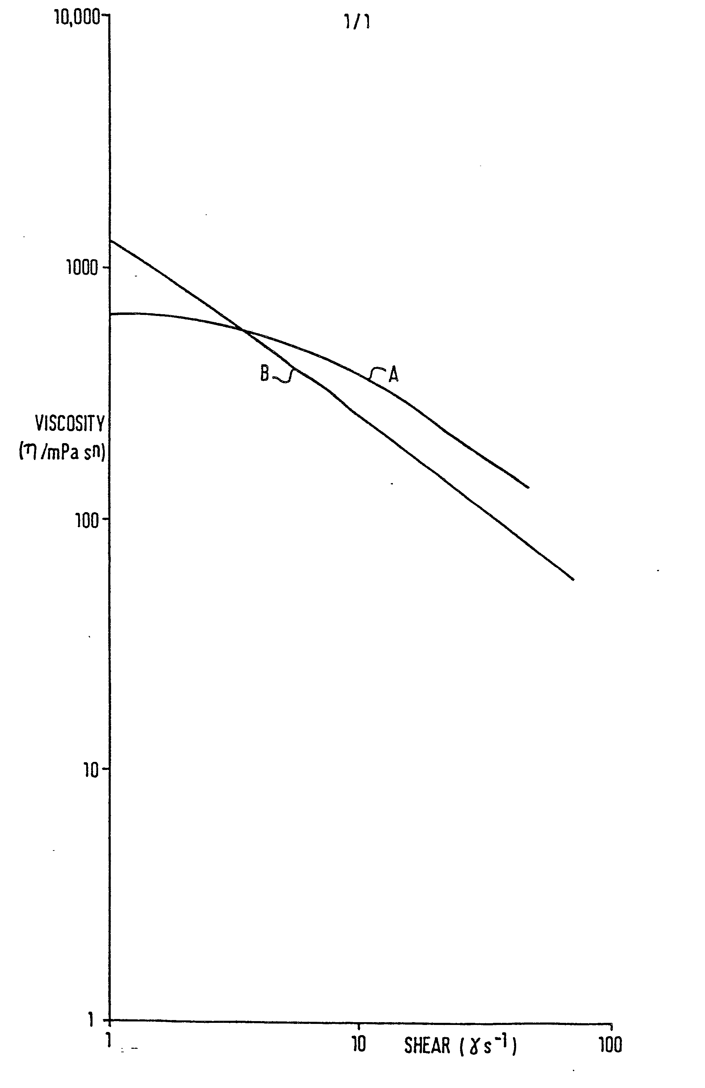

The figure accompanying this specification is a logarithmic plot of viscosity versus shear for the two solutions when determined under standardized conditions.

The viscosity of the present product is shown by the line A, whereas that for the conventional product is shown by line B.

Although the conventional product becomes noticeably less viscous with increasing shear, the present product tends to retain its viscosity. This reduced pseudoplasticity is desirable in thickening agents for certain end-uses, as mentioned previously.

16.

Example 2: Low viscosity gum

The procedure of Example 1 was repeated using another laboratory stock of the strain X. campestris ATCC 13951. The conditions were as in Example 1 except that the initial glucose concentration was 43 g/l and the pH was maintained at 6.8. The temperature of the medium was again held at $35^{o}C$ during the fermentation.

After 46 hours the fermentation was substantially complete, the residual glucose level being 4.6 g/l. and the final broth having a consistency index K of 4,465 and n of 0.31. The broth gave 13.3 g/l of total precipitable matter, TPM, which gave the product analysis shown in Table 8:

### Table 8

| | |
|---|---|
| Moisture | 6.31% |
| Ash | 13.0% |
| Nitrogen | 2.23% |
| Pyruvate | 1.94% |

The product at 1% per cent solution in water or 1% Na Cl solution had the viscosity characteristics shown in Table 9:

17.

Table 9

| Water | K | 8.85 |
|-------|---|------|
|       | n | 0.48 |
|       |   |      |
| 1% NaCl | K | 5.67 |
|         | n | 0.56 |

The inherent viscosity of the product was 8.6 dl/g.

A parallel fermentation under the same conditions except for a change of temperature to $30^{\circ}C$ gave a conventional xanthan gum product.

The results show that as in Example 1 the fermentation at an unusually high temperature leads to a low viscosity xanthan gum otherwise similar to a conventional xanthan gum.

Xanthan gums of the present invention can also be obtained using continuous culture and/or using other Xanthomonas spp, provided that the fermentation temperature is above $30^{\circ}C$.

Example 3

Water based paint

A paint with good flow characteristics is formulated as follows:-

| | Parts by weight |
|---|---|
| water | 168 |
| xanthan gum from Example 1 or Example 2 (3 - 5%) | 275 |
| potassium tripolyphosphate | 1 |
| propylene glycol | 30 |
| polyethoxylated nonyl phenol | 3 |
| phenyl mercuric acetate (fungicide) | 0.3 |
| titanium dioxide (pigment) | 200 |
| calcium carbonate (extender) | 100 |
| talc | 142 |
| defoaming agent (Nuodex AS-100) | 2 |
| vinyl acetate/ethyl acrylate (80/20) copolymer latex (55% solids) | 228 |
| butyl cellosolve acetate | 8 |

* (depending on viscosity)

Example 4

Acid dye printing paste for wool

(a) Xanthan paste

55g Xanthan from Example 1 or Example 2 was added gradually to 2 litres water containing formation (0.1% concentration) and stirred 3 hours till a smooth paste was obtained.

(b) Dye solution

| | |
|---|---|
| Lissamine Ultra Sky RS | 15 g |
| Glycerol | 75 g |
| Hot Water | 200 ml |

The components were mixed and brought to the boil and stirred until the dye solution (b) was added with stirring.


Example 5

Procion dye printing paste for viscose and cotton

| (a) | | |
|---|---|---|
| | Procion Brilliant Red H-8B | 50 g |
| | urea | 100 g |
| | water | 490 g |
| | Calgon | 2 g |

The components were mixed, brought to 70°C with stirring for complete dissolution of the dyestuff.

| (b) | | |
|---|---|---|
| | Xanthan Paste (Example 4(a)) | 350 g |
| | Resist salt L | 10 g |

The components were mixed well.

The dye solution (a) was added to mixture (b) and stirred well.

Just before use 25g sodium bicarbonate was stirred in.

Both pastes of Examples 4 and 5 give good performance in screen printing.

CLAIMS:

1.     A xanthan gum with an intrinsic viscosity $[\eta]$ of less than 14 dl/g.

2.     A xanthan gum according to claim 1 which has an intrinsic viscosity $[\eta]$ of less than 12.5 dl/g.

3.     A thickened aqueous composition when thickened using a xanthan gum according to claim 1 or claim 2.

4.     A composition according to claim 3 in the form of a water-based paint, a textile printing paste, or a cleaning composition.

5.     A method for producing a xanthan gum by fermentation of a carbohydrate-containing broth using a micro-organism of the genus Xanthomonas , characterized in that the fermentation is effected at a broth temperature of at least 31$^{\circ}$C and a xanthan gum with an intrinsic viscosity $[\eta]$ of less than 14 dl/g is produced.

6.     A method according to claim 5, wherein the fermentation is effected at a broth temperature of 32 to 38$^{\circ}$C.

7.     A method according to claim 6, wherein the fermentation is effected at a broth temperature of about 35$^{\circ}$C.

8. A method according to claim 5, 6 or 7, wherein the method is carried out by batch or continuous fermentation using a micro-organism of the species X. campestris, the broth containing a carbohydrate, an inorganic and/or organic source of assimilable nitrogen, other nutrients including trace elements, and any necessary essential growth factors.

9. A process according to any of claims 5 to 8, wherein after the fermentation the xanthan gum is isolated.

10. A method for the production of xanthan gum substantially as hereinbefore described in the examples 1 and 2.